# EUROPÄISCHE PATENTSCHRIFT

(11) **EP 3 797 692 B1**
(45) Veröffentlichungstag und Bekanntmachung des Hinweises auf die Patenterteilung: **18.01.2023**
(21) Anmeldenummer: 19200340.8
(22) Anmeldetag: 30.09.2019
(51) Int. Cl.: A61B 6/00, G06T 7/00, A61B 6/03, A61B 6/08, A61G 3/00

(54) **VERFAHREN UND VORRICHTUNG ZUR ANSTEUERUNG EINES MEDIZINISCHEN BILDGEBUNGSGERÄTS**
METHOD AND DEVICE FOR CONTROLLING A MEDICAL IMAGING DEVICE
PROCÉDÉ ET DISPOSITIF DE COMMANDE D'UN APPAREIL D'IMAGERIE MÉDICALE

(43) Veröffentlichungstag der Anmeldung: 31.03.2021
(73) Patentinhaber: Siemens Healthcare GmbH, 91052 Erlangen (DE)
(72) Erfinder: Lautenschläger, Stefan, 90762 Nürnberg (DE); Dr. Müller, Kerstin, San Francisco, CA-94109 (US)

(56) Entgegenhaltungen:
- US-A1- 2012 093 383
- US-A1- 2015 254 837
- US-A1- 2015 289 779
- US-A1- 2017 323 447
- US-A1- 2018 025 255
- US-A1- 2019 117 179
- US-A1- 2019 200 274

## Beschreibung

Die Erfindung betrifft ein Verfahren und eine Vorrichtung zur Ansteuerung eines medizinischen Bildgebungsgeräts mittels einer Steuerungseinheit für das Erzeugen eines zweiten, dreidimensionalen Bilddatensatzes, welcher einen Zielbereich in einem Untersuchungsbereich eines Patienten mit einer funktionellen Beeinträchtigung umfasst. Die Erfindung betrifft weiterhin ein Fahrzeug umfassend eine Vorrichtung zur Ansteuerung eines medizinischen Bildgebungsgeräts und ein Computerprogrammprodukt.

Für die Behandlung von einem Traumapatienten oder einen Schlaganfallpatienten wird eine Diagnose typischerweise durch einen Arzt und mit Hilfe von medizinischen Bilddatensätzen durchgeführt. Für eine vollständige Analyse der medizinischen Situation eines Patienten, werden häufig dreidimensionale Volumenbilddaten mittels eines Computertomographie-Geräts (CT-Geräts) breitgestellt. Das bedeutet, der Bildgebungsprozess besteht auf einer Mehrzahl an Röntgen-Projektionsmessdaten, welche aus unterschiedlichen Winkelbereichen aufgenommen werden und einer folgenden mathematischen Rücktransformation um die dreidimensionalen Volumenbilddaten basierend auf den Projektionsmessdaten zu rekonstruieren (z.B. mittels eines Rekonstruktionsalgorithmus der gefilterten Rückprojektion). Ausgehend von diesen dreidimensionalen Volumenbilddaten können auch zweidimensionale Schichtbilddatensätze, welche jeweils ein Schnittbild durch das abgebildete Volumen darstellen, erzeugt werden.

Im Falle eines schweren Traumas oder Schlaganfalls ist Zeit ein wichtiger Faktor. Je früher die richtige Behandlung beginnt, desto besser sind die Aussichten auf einen Behandlungserfolg, d.h. eine Vermeidung oder zumindest Reduzierung von bleibenden Schäden. Im Falle eines Schlaganfalls kann es beispielsweise äußerst wichtig für die Sauerstoffversorgung der Gehirnregionen sein, dass ein Thrombus möglichst bald entfernt wird. Eine detaillierte Diagnose und daher die Bereitstellung von möglichst aussagekräftigen Bilddatensätzen ist daher für die richtige Behandlung wesentlich.

Ein typisches Setup einer Bildgebungsreihe insbesondere bei einem Schlaganfallpatienten bzw. bei Verdacht auf einen Schlaganfall, besteht dabei häufig aus einem nativen CT-Bilddatensatz des Kopfes des Patienten ohne Kontrastmittelunterstützung, um andere Erkrankungen oder einen blutungsinduzierten Schlaganfall ausschließen zu können. Liegt ein ischämischer Infarkt vor, kann weiter ein CT-Angiographie-Bilddatensatz vorgesehen sein, um dasjenige Blutgefäß zu identifizieren, welches durch ein Blutgerinnsel verschlossen ist, d.h. welches von einem Gefäßverschluss betroffen ist. Außerdem wird häufig ein CT-Perfusions-Bilddatensatz erzeugt, um den ischämischen Kernbereich und diejenigen Bereiche des Gehirns, welche von einer verminderten Durchblutung betroffen sind, jedoch potenziell noch rettbar, sind, zu identifizieren. Basierend auf diesen Bilddaten kann dann entsprechend eine zielgerichtete Behandlungsentscheidung getroffen werden, beispielsweise ob lediglich eine Beobachtung, eine intravenöse Thrombolyse und/oder sogar eine mechanische Thrombektomie notwendig ist.

Eine möglichst zeiteffiziente Bereitstellung der Bilddatensätze, eine möglichst gute Abdeckung des betroffenen Bereiches durch den Bilddatensatz und ein reibungsloser Ablauf der Bilddatenerzeugung ist daher im Falle eines Schlaganfallpatienten besonders wünschenswert. Zeitgleich muss stets eine unnötige Strahlenbelastung vermieden werden.

US 2015/0289779 A1 betrifft ein System und ein Verfahren zur Diagnose von fokaler kortikaler Dysplasie. Dabei wird vorgeschlagen, Bilddaten eines Subjekts bei einer ersten Auflösung aufzunehmen, die aufgenommenen Bilddaten zur Bestimmung einer Dicke der grauen Substanz auszuwerten und die Ergebnisse für die Hemisphäre mit dem für die rechte Hemisphäre basierend auf entsprechenden geometrischen Merkmalen zu vergleichen. Die entstehende Unterschiedskarte wird genutzt, um Bereiche abnormer Differenzen zu identifizieren. In den Regionen abnormaler Unterschiede werden Bilddaten einer zweiten Auflösung aufgenommen und dargestellt.

US 2015/0254837 A1 betrifft Systeme und Verfahren zur Diagnose von Schlaganfällen. Dort wird vorgeschlagen eine Zeitsequenz von Computertomographiebildern nach Gabe eines Kontrastmittels aufzunehmen, woran ein Verfahren zur Ableitung von Informationen über den Ort und die Eigenschaften eines Blutgerinnsels ermittelt werden. Dies geschieht durch Markierung des Anfangs und des Endes durch einen Benutzer.

US 2019/0117179 A1 offenbart Systeme und Verfahren zur Wahl einer Handhabungsstrategie bei akuten ischämischen Schlaganfällen unter Nutzung von Rotationsangiographie. Dabei soll eine effiziente und quantitative Beurteilung der arteriellen Korreteralen innerhalb des Gehirns zur Unterstützung der Entscheidungen erfolgen. Dabei wird die Verwendung von Rotationsangiographie vorgeschlagen.

US 2018/0025255 A1 offenbart ein Klassifizierungsverfahren und eine Klassifizierungsvorrichtung, wobei ein Klassifizierer der künstlichen Intelligenz auf medizinische Bilddaten angewendet werden soll, nachdem diese in unterschiedliche Regionen unterteilt wurden.

US 2019/0200274 A1 betrifft ein Verfahren zur Bereitstellung von Bilddaten an eine zentrale Einheit. Dort wird ein Fahrzeug als mobile Schlaganfall-Einheit verwendet, welches eine mobile Bildgebungseinrichtung 5 enthält, so dass nach Übertragung von Bilddaten ein Speziallist an einem entfernten Ort die _Bilddaten beurteilen kann.

US 2017/0323447 A1 betrifft eine medizinische Bildaufnahmeeinrichtung und ein Verfahren. In einem Positionierbild werden Anteile des Subjekts identifiziert, woraufhin eine Diagnoseunterstützungsverarbeitung diesen Anteilen potentielle Diagnosen assoziieren kann. Danach können Bildgebungsbedingungen einer Hauptbildaufnahme bezüglich einer Läsion, die als Verarbeitungsergebnis der Diagnoseunterstützungsverarbeitung aufgefunden wurde, definiert werden, wobei dann die Hauptbildgebung unter Berücksichtigung der Bildgebungsbedingung in einem Bildbereich, der die Läsion enthält, erfolgt.

US 2012/0093383 A1 betrifft ein Verfahren zur sequentiellen Aufnahme von Bildern. Dabei werden Referenzbilddaten des Objekts durch ein Bildgebungssystem aufgenommen und es werden durch Registrierung mit einem Eingabebild Bildregionen identifiziert und zur Grundlage der Aufnahme weiterer Bilddaten des Objekts gemacht.

Aufgabe der Erfindung ist es daher ein verbessertes Verfahren und eine verbesserte Vorrichtung zur Ansteuerung eines medizinischen Bildgebungsgeräts für das Erzeugen eines dreidimensionalen Bilddatensatzes bereitzustellen.

Die Aufgabe wird gelöst durch die Merkmale der unabhängigen Patentansprüche. Weitere vorteilhafte und teils für sich erfinderische Ausführungsformen und Weiterbildungen der Erfindung sind in den Unteransprüchen und der nachfolgenden Beschreibung dargelegt.

Nachstehend wird die erfindungsgemäße Lösung der Aufgabe sowohl in Bezug auf das beanspruchte Verfahren als auch in Bezug auf die beanspruchte Vorrichtung beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf eine Vorrichtung gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Die Erfindung betrifft ein Verfahren zur Ansteuerung eines medizinischen Bildgebungsgeräts, insbesondere eines mobilen medizinischen Bildgebungsgeräts, mittels einer Steuerungseinheit für das Erzeugen eines zweiten, dreidimensionalen Bilddatensatzes, welcher einen Zielbereich in einem Untersuchungsbereich eines Patienten mit einer funktionellen Beeinträchtigung umfasst. Dafür weist das erfindungsgemäße Verfahren die folgenden Schritte auf:
- Bereitstellen eines ersten dreidimensionalen Bilddatensatzes umfassend den Untersuchungsbereich des Patienten mittels einer Datenverarbeitungseinheit,
- Identifizieren des Zielbereichs in dem ersten dreidimensionalen Bilddatensatz mittels der Datenverarbeitungseinheit, wobei ein Teilbereich des Untersuchungsbereichs bestimmt wird, welcher die funktionelle Beeinträchtigung aufweist,
- Bestimmen eines Bildgebungsparameters für die Erzeugung des zweiten dreidimensionalen Bilddatensatz basierend auf dem identifizierten Zielbereich mittels der Datenverarbeitungseinheit,
- Ansteuern des medizinischen Bildgebungsgeräts basierend auf dem Bildgebungsparameter für die Erzeugung des zweiten, dreidimensionalen Bilddatensatzes mittels der Steuerungseinheit,
- wobei bei Vorliegen von einer Mehrzahl an ersten dreidimensionale Bilddatensätzen, derjenige erste dreidimensionale Bilddatensatz bereitgestellt wird, welcher den geringsten zeitlichen Abstand zum Zeitpunkt der Bereitstellung aufweist.

Insbesondere kann mittels des medizinischen Bildgebungsgeräts ein dreidimensionaler Bilddatensatz (3D Bilddatensatz), beispielsweise ein Computertomographie-Bilddatensatz (CT-Bilddatensatz) erzeugt werden. Das Erzeugen eines 3D Bilddatensatzes kann dabei die Bildakquisition, d.h. die Aufnahme der Messdaten mittels einer Messdatenaufnahmeeinheit des medizinischen Bildgebungsgeräts, umfassen. Die Messdatenaufnahmeeinheit umfasst beispielsweise eine Röntgenquelle und einen Röntgendetektor, welche für die Aufnahme von Messdaten relativ zum Patienten positioniert wird. Das Erzeugen kann daneben auch die Bildrekonstruktion des 3D Bilddatensatzes auf Basis von aufgenommenen Messdaten umfassen.

Ein 3D Bilddatensatz erlaubt eine dreidimensionale, insbesondere räumlich dreidimensionale, Darstellung des Untersuchungsbereichs des Patienten. Ein 3D Bilddatensatz kann auch als eine Mehrzahl an Schichtbilddatensätzen dargestellt werden. Ein Schichtbilddatensatz umfasst jeweils eine Schicht des 3D Bilddatensatz an einer Position entlang einer ausgezeichneten Achse. Ein Schichtbilddatensatz erlaubt dann jeweils eine zweidimensionale, insbesondere räumlich zweidimensionale, Darstellung der jeweiligen Schicht.

Vorteilhafterweise umfasst der 3D Bilddatensatz mehrere Voxel, insbesondere Bildpunkte. Dabei kann jedes Voxel vorzugsweise jeweils einen Wert, insbesondere einen Bildwert, aufweisen, beispielsweise einen Grauwert und/oder einen RGB-Farbwert und/oder einen Intensitätswert. Analog dazu kann ein Schichtbilddatensatz mehrere Pixel, insbesondere Bildpunkte, umfassen. Dabei kann jeder Pixel vorzugsweise jeweils einen Wert, insbesondere einen Bildwert, aufweisen, beispielsweise einen Grauwert und/oder einen RGB-Farbewert und/oder einen Intensitätswert.

Das medizinisches Bildgebungsgerät kann beispielsweise ein CT-Gerät oder ein C-Bogenröntgengerät umfassen. Es sind auch andere Bildgebungsgeräte denkbar, welche ausgebildet sind einen 3D Bilddatensatz zu erzeugen.

Der Patient kann ein tierischer Patient und/oder ein menschlicher Patient sein. Ferner kann der Untersuchungsbereich des Patienten einen anatomischen und/oder räumlichen Bereich des Patienten umfassen, der einen vorbestimmten Gewebebereich und/oder einen für eine Diagnose notwendigen räumlichen Bereich umfasst. Der Untersuchungsbereich kann dabei einen Körperbereich, beispielsweise den Kopf, den Thorax oder ein Arm umfassen. Der Untersuchungsbereich kann gegebenenfalls auch den gesamten Körper des Patienten umfassen.

Der bereitgestellte erste 3D Bilddatensatz umfasst insbesondere den Untersuchungsbereich des Patienten. Das Bereitstellen des ersten 3D Bilddatensatzes kann beispielsweise ein Erfassen und/oder Auslesen eines computerlesbaren Datenspeichers und/oder ein Empfangen aus einer Speichereinheit umfassen. Ferner kann das erfindungsgemäße Verfahren auch das Erzeugen des ersten 3D Bilddatensatz mittels des medizinischen Bildgebungsgeräts umfassen, welcher anschließend für die weiteren Schritte des Verfahrens bereitgestellt werden kann.

Insbesondere kann der erste 3D Bilddatensatz von dem gleichen medizinischen Bildgebungsgerät erzeugt werden bzw. erzeugt worden sein wie der zweite 3D Bilddatensatz. Der erste 3D Bilddatensatz kann dabei jedoch mittels eines anderen Aufnahmemodus des medizinischen Bildgebungsgeräts erzeugt werden bzw. worden sein als der zweite 3D Bilddatensatz. Beispielsweise ist der erste 3D Bilddatensatz ein nativer CT-Bilddatensatz oder ein CT-Angiographie-Bilddatensatz und der zweite 3D Bilddatensatz ein CT-Perfusions-Bilddatensatz. Es sind auch anderweitige Aufnahmemodi bzw. Bilddatensätze denkbar

Gemäß dem erfindungsgemäßen Verfahren umfasst der zweite 3D Bilddatensatz einen Zielbereich des Untersuchungsbereichs eines Patienten mit einer funktionellen Beeinträchtigung. Eine funktionelle Beeinträchtigung kann dabei bedeuten, dass die normale, körperliche Funktion einer Funktionseinheit des Körpers des Patienten, welche von dem Untersuchungsbereich umfasst ist, gestört oder eingeschränkt ist. Eine Funktionseinheit kann beispielsweise ein Organ, ein Knochen, ein Blutgefäß oder ähnliches sein. In einem konkreten Beispiel eines Schlaganfallpatienten ist die Funktionseinheit etwa das Gehirn eines Patienten.

Der vom Untersuchungsbereich umfasste Zielbereich kann dann zumindest den Teilbereich des Untersuchungsbereichs umfassen, welcher die funktionelle Beeinträchtigung aufweist, d.h. in welchem die funktionelle Beeinträchtigung basierend auf dem ersten 3D Bilddatensatz lokalisiert werden kann. Das kann bedeuten, der Zielbereich kann zumindest den Teilbereich des Untersuchungsbereichs umfassen, mit welchem die funktionelle Beeinträchtigung im ersten 3D Bilddatensatz assoziiert, d.h. verknüpft, werden kann. Das kann bedeuten, dass der Zielbereich kann eine von der funktionellen Beeinträchtigung betroffene Funktionseinheit, eine Untereinheit der betroffenen Funktionseinheit, auf welche die funktionelle Beeinträchtigungen eingeschränkt werden kann, oder auch einen konkreten Ursprungsort der funktionellen Beeinträchtigung innerhalb der Funktionseinheit umfasst.

Der Schritt des Identifizierens des Zielbereichs kann dann ein Lokalisieren des Teilbereichs des Untersuchungsbereichs im ersten 3D Bilddatensatz umfassen, welcher basierend auf dem ersten 3D Bilddatensatz mit der funktionellen Beeinträchtigung verknüpft werden kann. Das heißt, es können im ersten 3D Bilddatensatz die Bildbereiche identifiziert werden, welche dem Teilbereich entsprechen. Es kann auch das Lokalisieren eines Orts, d.h. eines Auftrittsorts oder eines Ursprungsorts, der funktionellen Beeinträchtigung im Untersuchungsbereich basierend auf dem ersten 3D Bilddatensatz umfassen. Das heißt, der Schritt des Identifizierens kann beispielsweise umfassen, eine von der funktionellen Beeinträchtigung betroffene Funktionseinheit, eine Untereinheit der betroffenen Funktionseinheit, auf welche das Auftreten der funktionelle Beeinträchtigungen eingeschränkt werden kann, oder auch einen konkreten Ursprungsort der funktionellen Beeinträchtigung innerhalb der Funktionseinheit basierend auf dem ersten 3D Bilddatensatz zu lokalisieren bzw. zu bestimmen.

Der identifizierte Zielbereich kann im ersten 3D Bilddatensatz als ein dreidimensionales Volumen identifiziert werden.

Er kann auch als zweidimensionales Areal in einem oder jeweils in mehreren Schichtbilddatensätzen des ersten 3D Bilddatensatz identifiziert werden. Der identifizierte Zielbereich kann auch durch einzelne oder eine Mehrzahl von Pixel- bzw. Voxelkoordinaten im ersten 3D Bilddatensatz oder einer seiner Schichtbilddatensätze bestimmt sein. Beispielsweise wird der identifizierte Zielbereich in einer Darstellung des ersten 3D Bilddatensatzes für einen Anwender optisch hervorgehoben. Beispielsweise wird eine Markierung in Form einer dreidimensionalen oder zweidimensionalen Box im Bilddatensatz den Bilddaten überlagert dargestellt, welche den identifizierten Zielbereich im ersten 3D Bilddatensatz markiert. Derart kann gegebenenfalls eine Überprüfung des identifizierten Zielbereichs durch einen Anwender ermöglicht werden.

Ein Beispiel einer funktionellen Beeinträchtigung liegt etwa bei einer Sauerstoff- und Glukoseunterversorgung eines Gehirnbereichs nach einem Gefäßverschluss einer Gehirnarterie vor, d.h. einem Perfusionsdefizit im Rahmen eines ischämischen Infarktes. Der Zielbereich kann dann das Gehirn als Funktionseinheit umfassen. Das heißt, beim Identifizieren können die mit dem Gehirn des Patienten assoziierten Bildbereiche im ersten 3D Bilddatensatz bestimmt werden. Der Zielbereich kann auch den Teil des Gehirns als Untereinheit der betroffenen Funktionseinheit umfassen, welcher im ersten 3D Bilddatensatz als vom Perfusionsdefizit betroffen oder mit diesem verknüpft bestimmt werden kann. Das heißt, beim Identifizieren können die Bildbereiche des ersten 3D Bilddatensatz bestimmt werden, welche mit dem Perfusionsdefizit verknüpft werden können. Der Zielbereich kann den ischämischen Kernbereich selbst umfassen, welcher das Zentrum des Perfusionsdefizits darstellt. Hier beträgt der Blutfluss maximal noch 20% der normalen Perfusionsmenge, was in kürzester Zeit zum irreversiblen Funktionsverlust führt. In der, den Kernbereich umgebenden Penumbra, auch Kernschatten genannt, ist dagegen aufgrund einer Restdurchblutung das Gehirngewebe eine Erholung des Gewebes möglich, sofern zeitnah die entsprechende Behandlung des Patienten bereitgestellt wird. Das heißt, beim Identifizieren können die Bildbereiche im ersten 3D Bilddatensatz bestimmt werden, welche mit dem ischämischen Kernbereich verknüpft werden können.

Ein anderes Beispiel einer funktionellen Beeinträchtigung liegt beispielsweise bei einem Knochenbruch eines Skelettknochens vor. Der Zielbereich umfasst dann beispielsweise zumindest den Bereich der Bruchstelle. Der Zielbereich kann jedoch auch beispielsweise den gesamten, betroffenen Knochen oder den Körperabschnitt des Patienten umfassend den Knochen umfassen. Ein weiteres Beispiel einer funktionellen Beeinträchtigung liegt beispielsweise bei einem Gefäßverschluss eines Blutgefäßes abseits des Gehirns vor, beispielsweise am Herzen. Der Zielbereich umfasst dann beispielsweise zumindest den Ort oder den Gefäßabschnitt, an welchem der Gefäßverschluss vorliegt. Darüber hinaus kann es noch andere Anwendungen des erfindungsgemäßen Verfahrens geben.

Darüber hinaus ist weiterhin denkbar, dass das Identifizieren auch das Bestimmen einer Ausdehnung der funktionellen Beeinträchtigung oder eines Volumens eines von der funktionellen Beeinträchtigung betroffenen Bereichs umfasst, beispielsweise eine maximale Ausdehnung entlang zumindest einer Raumdimension des 3D Bilddatensatzes.

Das Identifizieren kann im ersten 3D Bilddatensatz und/oder basierend auf Schichtbilddatensätzen des 3D Bilddatensätzen erfolgen. Das Identifizieren kann dabei insbesondere durch eine Analyse bzw. Weiterverarbeitung der Bildwerte oder durch sie abgebildete Strukturen im ersten 3D Bilddatensatz oder seiner Schichtbilddatensätze beruhen.

Der basierend auf dem Zielbereich bestimmte Bildgebungsparameter, kann ein Bildgebungsparameter betreffend die Bildakquisition und/oder die Bildrekonstruktion umfassen. Der bestimmte Bildparameter kann insbesondere darauf abzielen, die Erzeugung des zweiten 3D Bilddatensatzes auf den identifizierten Zielbereich, d.h. insbesondere den durch den mittels dem ersten 3D Bilddatensatz identifizierten Zielbereich definierten Teilbereich des Untersuchungsbereichs, welcher die funktionelle Beeinträchtigung aufweist, abzustimmen. Dadurch kann erreicht werden, dass der Bereich mit der funktionellen Einschränkung optimal mittels des zweiten 3D Bilddatensatz dargestellt werden kann.

Es kann auch mehr als ein Bildgebungsparameter bestimmt werden. Beispielsweise kann ein Aufnahmebereich, auch Scanbereich genannt, basierend auf dem Zielbereich festlegen werden. Der Aufnahmebereich kann dem Teil des Patienten oder des Untersuchungsbereichs entsprechen, für welchen Messdaten mittels des medizinischen Bildgebungsgeräts für die Erzeugung des zweiten, 3D Bilddatensatz aufgenommen werden sollen. Der Bildgebungsparameter kann einen Positionierungsparameter des medizinischen Bildgebungsgeräts, insbesondere dessen Messdatenaufnahmeeinheit, relativ zu dem Patienten oder relativ zum Untersuchungsbereich, umfassen. Der Bildgebungsparameter kann auch ein Rekonstruktionsvolumen für die Rekonstruktion des 3D Bilddatensatzes oder auch ein anderweitiger Rekonstruktionsparameter umfassen. Darüber hinaus sind auch andere Bildgebungsparameter denkbar.

Der Schritte des Identifizierens und/oder des Bestimmens kann dabei vollautomatisch und ohne weiteres Zutun eines Anwenders mittels der Datenverarbeitungseinheit durchgeführt werden. Es kann jedoch auch eine Interaktion mit einem Anwender, beispielsweise eine Bestätigung oder eine Korrekturmöglichkeit für den Zielbereich oder den Bildgebungsparameter oder ähnliches durch einen Anwender vorgesehen sein. Dazu kann insbesondere eine Eingabeeinheit vorgesehen sein, welche ausgebildet ist Eingaben eines Anwenders in Form von Berührung, Gesten, Sprache oder ähnliches in für die Datenverarbeitung interpretierbare Anweisungen umzusetzen.

Basierend auf dem bestimmten Bildgebungsparameter wird erfindungsgemäß das medizinische Bildgebungsgerät mittels der Steuerungseinheit angesteuert. Dazu kann der Bildgebungsparameter beispielsweise mittels einer Schnittstelle an die Steuereinheit des medizinischen Bildgebungsgeräts ausgegeben werden. Der Bildgebungsparameter kann vor oder auch nach der Ausgabe an die Steuereinheit in eine Steuerungsanweisung für das medizinische Bildgebungsgerät übersetzt werden, welche von der Steuerungseinheit für die Ansteuerung des Bildgebungsprozesses, d.h. der Erzeugung des zweiten 3D Bilddatensatzes, interpretierbar ist. Analog zu dem bestimmten Bildgebungsparameter kann das Ansteuern mittels der Steuereinheit das Ansteuern der Bildakquisition und/oder das Ansteuern der Bildrekonstruktion umfassen.

Der Schritt des Ansteuerns kann dabei vollautomatisch und ohne weiteres Zutun eines Anwenders mittels der Steuerungseinheit basierend auf dem bestimmten Bildgebungsparameter durchgeführt werden. Es kann jedoch auch eine Interaktion mit einem Anwender, beispielsweise eine Bestätigung, ein Starten oder Stoppen, eine Korrekturmöglichkeit oder ähnliches durch einen Anwender vorgesehen sein.

Der zweite 3D Bilddatensatz kann insbesondere einen Teilausschnitt des ersten, 3D Bilddatensatzes darstellen, welcher zumindest den Zielbereich umfasst. Es kann auch Fälle geben, in welchen der zweite, 3D Bilddatensatz den gleichen Bildausschnitt darstellt wie der erste 3D Bilddatensatz, sofern beispielsweise ein derartiger Aufnahmebereich als Bildgebungsparameter mittels des erfindungsgemäßen Verfahrens bestimmt ist.

Vorzugweise wird der zweite 3D Bilddatensatz in zeitlicher Nähe zum ersten 3D Bilddatensatz erzeugt. Besonders bevorzugt wird dabei eine Bewegung des Patienten, insbesondere des Untersuchungsbereichs, zwischen dem ersten und dem zweiten Bilddatensatz weitestgehend vermieden.

Die Erfinder haben erkannt, dass in vielen Fällen einer Bildgebungsprozedur in zeitlicher Nähe aufeinanderfolgend zumindest zwei Bilddatensätze aufgenommen werden, so dass auf einen bereits vorliegenden ersten Bilddatensatz zurückgegriffen werden kann, um den Bildgebungsprozess für das Erzeugen des nachfolgenden, zweiten Bilddatensatz zu optimieren und möglichst auch zu automatisieren. Insbesondere ist dies der Fall, wenn verschiedene Bildmodalitäten, beispielsweise ein nativer CT-Bilddatensatz und ein CT-Angiographie-Bilddatensatz oder ein CT-Perfusions-Bilddatensatz in kurzer zeitlicher Abfolge und vorzugsweise ohne eine Umlagerung des Patienten durchgeführt und für eine umfängliche Diagnose kombiniert werden sollen. Vorteilhaft kann mittels des Verfahrens ein besonders zeiteffizienter Arbeitsablauf ermöglicht werden. Insbesondere können zeitaufwendige und fehleranfällige manuelle Schritte und Anpassungen für die Erzeugung des zweiten 3D Bilddatensatz reduziert werden. Ebenso kann das Verfahren vorteilhaft zu einer Dosisminimierung beitragen, indem ein Bildgebungsparameter gewählt wird, beispielsweise ein optimierter Aufnahmebereich, wodurch eine unnötige Strahlenbelastung vermieden werden kann und indem Wiederholungen von Aufnahmen reduziert werden können.

Für besonders zeitkritische Anwendungen, beispielsweise bei einem Traumapatienten oder Schlaganfallpatienten, ermöglichen momentane Entwicklungen außerdem flexiblere Einsätze von CT-Geräten, beispielsweise in Krankenwägen (sogenannte "mobile stroke units", mobile Schlaganfalleinheiten) oder sogar in Helikoptern, welche eine noch zeitnähere Untersuchung eines Patienten ermöglichen. Diese leiden jedoch, beispielsweise durch das eingeschränkte Platzangebot, meist unter Einschränkungen. Es kann etwa nur ein eingeschränkter Aufnahmebereich zur Verfügung gestellt werden. In diesen Fällen besteht neben dem besonders reibungslosen Ablauf außerdem die Notwendigkeit einer möglichst optimalen Selektion des relevanten Körperbereichs für die Erzeugung eines aussagekräftigen Bilddatensatzes hinzu. Vorteilhaft kann mittels des erfindungsgemäßen Verfahrens sichergestellt werden, dass ein relevanter Teilbereich optimal selektiert und mittels des zweiten 3D Bilddatensatz abgebildet werden kann.

In einer Variante des erfindungsgemäßen Verfahrens umfasst der Untersuchungsbereich zumindest einen Teil des Kopfes des Patienten und der Zielbereich zumindest einen Teil des Gehirns des Patienten.

Der Zielbereich kann das ganze Gehirn oder nur ein Teil des Gehirns umfassen. Der Patient kann ein Traumapatient mit potenziellen Verletzungen oder Blutungen am Gehirn sein. Insbesondere kann der Patient ein Schlaganfallpatient aufweisend einen ischämischen Infarkt sein.

Bei dem Auftreten von funktionellen Beeinträchtigungen, beispielsweise hervorgerufen durch einen ischämischen Infarkt, das Gehirn betreffend ist in der Regel eine besonders zeitnahe und umfassende Diagnose wesentlich für eine optimale Behandlung des Patienten, so dass hier das erfindungsgemäße Verfahren besonders vorteilhaft eingesetzt werden kann. Darüber hinaus ist in diesen Fällen häufig nach einem ersten, meist nativen Bilddatensatz ohne Zugabe von Kontrastmitteln, die Kombination zumindest mit einem zweiten Aufnahmemodus, beispielsweise einem CT-Angiographie-Bilddatensatz oder einem CT-Perfusions-Bilddatensatz, für die Diagnose vorgesehen, so dass vorteilhaft in der Regel ein erster dreidimensionaler Bilddatensatz bereits vorliegt, auf den für die Ansteuerung des medizinischen Bildgebungsgeräts zum Erzeugen des zweiten 3D Bilddatensatzes zurückgegriffen werden kann. Eine zeiteffiziente exakte Bestimmung eines Zielbereichs und eine darauf abgestimmte Ansteuerung kann insbesondere wesentlich für eine erfolgreiche Erzeugung des zweidimensionalen Bilddatensatzes sein, wenn der zeitkritische Anwendungsfall mit Einschränkungen des medizinischen Bildgebungsgerät einhergeht.

Weiterhin ist in einer Ausgestaltung des Verfahrens vorgesehen, dass der Patient ein Schlaganfallpatient ist und im Schritt des Identifizierens des Zielbereichs als Teilbereich ein Teil des Gehirns des Patienten bestimmt wird, welcher von einem ischämischen Infarkt betroffen ist.

Ein Schlaganfall, d.h. insbesondere ein ischämischer Infarkt, stellt eine besonders zeitkritische Anwendung dar, wobei eine möglichst schnelle und umfassende Diagnose für das Ausmaß der Folgebeeinträchtigungen und das Überleben der Patienten wesentlich ist, so dass hier das erfindungsgemäße Verfahren besonders vorteilhaft eingesetzt werden kann.

Die Bestimmung des Teilbereichs kann dabei insbesondere durch eine Analyse bzw. Weiterverarbeitung der auftretenden Bildwerte oder durch sie erzeugte Strukturen im ersten 3D Bilddatensatz oder seiner Schichtbilddatensätze beruhen.

Beispielsweise kann das Gehirn eines Patienten basierend auf dem ersten 3D Bilddatensatz in verschiedene Gehirnareale unterteilt werden. Die Gehirnareale können beispielsweise, müssen jedoch nicht notwendigerweise, mit bestimmten Körperfunktionen verknüpft sein. Das Gehirn kann beispielsweise basierend auf einem anatomischen Atlas in die Gehirnareale unterteilt werden. Beispielsweise können die Gehirnareale jeweils bewertet werden hinsichtlich einer Wahrscheinlichkeit einer Betroffenheit durch den ischämischen Infarkt. Ein jeweiliges Gehirnareal kann teilweise oder ganz von dem ischämischen Infarkt betroffen sein. Der Zielbereich kann dann entsprechend zumindest ein als betroffen bewertetes Gehirnareal umfassen. Der Zielbereich kann auch mehr als ein als betroffen bewertetes Gehirnareal betreffen. Die Bewertung kann dabei auf der Analyse oder Weiterverarbeitung der Bildwerte beruhen.

Der Teilbereich, welcher von einem ischämischen Infarkt betroffen ist, kann auch unabhängig von einer vorherigen Unterteilung in verschiedene Gehirnareale, bestimmt werden, indem die auftretenden Bildwerte oder durch sie erzeugte Strukturen im ersten 3D Bilddatensatz oder seiner Schichtbilddatensätze analysiert oder weiterverarbeitet werden.

Ein betroffener Teilbereich kann beispielsweise im ersten 3D Bilddatensatz bzw. in einer jeweiligen Schichtbilddatensatz des ersten 3D Bilddatensatzes lokal oder global Bildwerte, örtlich zusammenhängende Gruppen von Bildwerten, einen durchschnittlichen Bildwert oder ähnliches aufweisen, welche bzw. welcher beispielsweise von einem Erwartungswert abweichen bzw. abweicht. Ein erwarteter Bildwert kann dabei basierend auf nicht betroffenen Gehirnarealen des Patienten, beispielsweise durch einen Vergleich der rechten und linken Hemisphäre des Gehirns, oder auch auf Erfahrungswerten aus bereits zuvor aufgenommenen Bilddatensätzen des Patienten selbst oder einer Vielzahl von Patienten beruhen.

Der bestimmte Teilbereich kann dabei ein dreidimensionales Volumen im ersten 3D Bilddatensatz darstellen oder, bei einer schichtweisen Betrachtung des ersten 3D Bilddatensatzes, eine zweidimensionale Fläche in einer Schicht des ersten 3D Bilddatensatzes.

In einer besonders vorteilhaften Ausgestaltung des Verfahrens wird im Schritt des Identifizierens ein ischämische Kernbereich im ersten 3D Bilddatensatz bestimmt.

Der ischämische Kernbereich umfasst den Bereich mit einer irreversiblen Schädigung des Gehirns durch das Perfusionsdefizit dar. Der ischämische Kernbereich liegt in der Regel im Zentrum eines durch ein Perfusionsdefizit betroffenen Gehirnareals und ist umgeben von der Penumbra, in der eine Restdurchblutung vorhanden ist. Der ischämische Kernbereich kann damit besonders vorteilhaft für eine Lokalisierung und Bestimmung des relevanten Bereichs genutzt werden. Die Bestimmung des ischämischen Kernbereichs kann außerdem gegebenenfalls Rückschlüsse über Ursprung, Ausmaß und Schaden durch den ischämischen Infarkt zulassen.

Eine Variante des erfindungsgemäßen Verfahrens sieht vor, dass der Zielbereich basierend auf einem Segmentationsalgorithmus oder einem Schwellwertverfahren automatisch im ersten 3D Bilddatensatz identifiziert wird.

Der Segmentationsalgorithmus kann beispielsweise auf einer pixelbasierten, voxelbasierten, kantenbasierten, flächenbasierten und/oder regionenbasierten Segmentierung basieren. Der Segmentierung kann auch ein Modellbasiertes, atlasbasiertes Verfahren zu Grunde liegen, wobei Annahmen über das zu segmentierende Objekt eingehen. Die Segmentierung kann dabei schichtweise, d.h. ausgehend von den zweidimensionalen Schichtbildern vorgehen oder es kann auch ein dreidimensionales Segmentierungsverfahren eingesetzt werden. Dabei kann der Schritt des Segmentierens auch halbautomatisiert umgesetzt sein. Beispielsweise können Startpunkte oder Keimzellen oder eine grobe Kontureninformation für die Segmentierung manuell gesetzt werden.

Mittels eines Segmentationsalgorithmus können vorteilhaft zusammenhängende Strukturen identifiziert werden. Beispielsweise kann das Gehirn, Gehirnareale oder vom ischämischen Infarkt betroffenen Teilbereiche des Gehirns segmentiert werden.

Ein Schwellwertverfahren kann umfassen, dass Bildwerte des ersten 3D Bilddatensatzes, beispielsweise gegeben in HU ("Hounsfield Units"), mit einem Schwellwert verglichen werden. Darauf basierend kann beispielsweise eine Unterscheidung getroffen werden, ob der Pixel oder Voxel aufweisend den Bildwert dem Zielbereich zuzuordnen ist oder nicht. Der Schwellwert kann dabei einem bereits zuvor beschriebenen Erwartungswert entsprechen. Ein Segmentationsalgorithmus kann auf einem Schwellwertverfahren basieren oder diesem vorausgehen, wobei Bildbereiche aufweisend einen Bildwert unter bzw. über dem vorgegebenen Schwellwert einem zu segmentierenden Bereich zugeordnet werden oder der Bilddatensatz für eine Segmentierung mittels eine Schwellwertverfahrens vorbereitet wird.

Dabei kann vorgesehen sein, dass der Schritt des Identifizierens das Anwenden einer trainierten Funktion umfasst.

Eine trainierte Funktion kann eingesetzt werden, um automatisch und zeiteffizient den Zielbereich zu bestimmen. Eine trainierte Funktion kann beispielsweise für ein Lokalisieren, ein Segmentieren und/oder ein Bewerten von Gehirnarealen eingesetzt werden. Eine trainierte Funktion kann eingesetzt werden um gegebenenfalls sogar den Bildgebungsparameters basierend auf dem Zielbereich abzuleiten.

Dabei kann die trainierte Funktion vorteilhafterweise durch ein Verfahren des maschinellen Lernens trainiert sein. Insbesondere kann die trainierte Funktion ein neuronales Netzwerk, insbesondere ein faltendes neuronales Netzwerk (engl. convolutional neural network, CNN) bzw. ein Netzwerk umfassend eine Faltungsschicht (engl. convolutional layer) sein.

Eine trainierte Funktion bildet Eingabedaten auf Ausgabedaten ab. Hierbei können die Ausgabedaten insbesondere weiterhin von einem oder mehreren Parametern der trainierten Funktion abhängen. Der eine oder die mehreren Parameter der trainierten Funktion können durch ein Training bestimmt und/oder angepasst werden. Das Bestimmen und/oder das Anpassen des einen oder der mehreren Parameter der trainierten Funktion kann insbesondere auf einem Paar aus Trainingseingabedaten und zugehörigen Trainingsausgabedaten basieren, wobei die trainierte Funktion zur Erzeugung von Ausgabedaten auf die Trainingseingabedaten angewendet wird. Insbesondere kann das Bewerten auf einem Vergleich der erzeugten Ausgabedaten und der Trainingsausgabedaten basieren. Im Allgemeinen wird auch eine trainierbare Funktion, d.h. eine Funktion mit noch nicht angepassten einen oder mehreren Parametern, als trainierte Funktion bezeichnet.

Andere Begriffe für trainierte Funktion sind trainierte Abbildungsvorschrift, Abbildungsvorschrift mit trainierten Parametern, Funktion mit trainierten Parametern, Algorithmus basierend auf künstlicher Intelligenz, Algorithmus des maschinellen Lernens. Ein Beispiel für eine trainierte Funktion ist ein künstliches neuronales Netzwerk, wobei die Kantengewichte des künstlichen neuronalen Netzwerks den Parametern der trainierten Funktion entsprechen. Anstatt des Begriffs "neuronales Netzwerk" kann auch der Begriff "neuronales Netz" verwendet werden. Insbesondere kann eine trainierte Funktion auch ein tiefes künstliches neuronales Netzwerk sein (engl. deep neural network, deep artificial neural network). Ein weiteres Beispiel für eine trainierte Funktion ist eine "Support Vector Machine", weiterhin sind auch insbesondere andere Algorithmen des maschinellen Lernens als trainierte Funktion einsetzbar. Die trainierte Funktion kann beispielsweise mittels einer Rückpropagation trainiert sein. Zunächst können Ausgabedaten durch Anwendung der trainierten Funktion auf Trainingseingabedaten bestimmt werden. Hiernach kann eine Abweichung zwischen den erzeugten Ausgabedaten und den Trainingsausgabedaten durch Anwendung einer Fehlerfunktion auf die erzeugten Ausgabedaten und die Trainingsausgabedaten ermittelt werden. Ferner kann zumindest ein Parameter, insbesondere eine Gewichtung, der trainierten Funktion, insbesondere des neuronalen Netzwerks, basierend auf einem Gradienten der Fehlerfunktion bezüglich des zumindest einen Parameters der trainierten Funktion iterativ angepasst werden. Hierdurch kann die Abweichung zwischen den Trainingsabbildungsdaten und den Trainingsausgabedaten während des Trainings der trainierten Funktion vorteilhafterweise minimiert werden.

Die Trainingseingabedaten können beispielweise eine Vielzahl an 3D Trainingsbilddatensätze aufweisend eine funktionelle Beeinträchtigung umfassen. Die Trainingsausgabedaten können beispielweise Trainingsbilddatensätze aufweisend lokalisierte Teilbereiche oder verknüpfte Zielbereichen, Bewertungen von Gehirnarealen oder Bildgebungsparameter umfassen.

Durch Anwenden eines künstlichen Intelligenzsystems, d.h. einer trainierten Funktion, können alle relevanten Einflussgrößen für das Identifizieren berücksichtigt werden, auch solche, für die ein Anwender keinen Zusammenhang zum Identifizieren abschätzen kann. Insbesondere kann mittels einer trainierten Funktion nach der Trainingsphase eine Identifizierung besonders verlässlich und zeiteffizient automatisiert ermöglicht werden.

In einer Ausgestaltung des Verfahrens ist der Bildgebungsparameter ein Bildakquisitionsparameter für die Aufnahme von Messdaten für das Erzeugen des zweiten, 3D Bilddatensatzes und/oder ein Bildrekonstruktionsparameter für die Bildrekonstruktion des zweiten 3D Bilddatensatzes umfasst.

Vorteilhaft kann eine Automatisierung oder zumindest Teilautomatisierung des Bildgebungsprozesses, d.h. des Erzeugens ermöglicht werden. Vorteilhaft kann eine Optimierung des Bildgebungsprozesses durch Vermeidung fehleranfälliger manueller Schritte und durch eine verbesserte Zeiteffizienz erreicht werden.

Ein Bildakquisitionsparameter kann dabei im Wesentlichen jeden Parameter die Messdatenaufnahme betreffend umfassen. Beispielsweise kann der Bildgebungsparameter ein Aufnahmebereich betreffen. Der Aufnahmebereich kann festlegen, welcher Bereich des Untersuchungsbereichs für die Erzeugung des zweiten 3D Bilddatensatz mittels des medizinischen Bildgebungsgeräts abgetastet werden soll. Er kann außerdem auch andere Parameter für die Messdatenaufnahme umfassen. Beispielsweise Einstellungsparameter der Messdatenaufnahmeeinheit, beispielsweise eine Einstellung der Röntgenquelle oder des Röntgendetektors. Der Bildgebungsparameter kann außerdem ein Positionierungsparameter der Messdatenaufnahmeeinheit relativ zum Patienten oder ein Bewegungsparameter der Messdatenaufnahmeeinheit relativ zum Patienten, beispielsweise eine Geschwindigkeit der Bewegung, betreffen.

Besonders bevorzugt umfasst der bestimmte Bildgebungsparameter zumindest einen Aufnahmebereich.

Vorteilhaft kann ein optimierter Aufnahmebereich, welcher insbesondere auch die Gegebenheiten des medizinischen Bildgebungsgeräts einbeziehen kann, bereitgestellt werden. Vorteilhaft kann ein dosisoptimierter Aufnahmebereich bereitgestellt werden.

Weiterhin kann das erfindungsgemäße Verfahren umfassen, dass der bestimmte Bildgebungsparameter ein Aufnahmebereich für die Aufnahme von Messdaten mittels des medizinischen Bildgebungsgeräts für das Erzeugen des zweiten, 3D Bilddatensatzes und das Ansteuern ein Positionieren einer Messdatenaufnahmeeinheit des medizinischen Bildgebungsgeräts relativ zum Patienten basierend auf dem bestimmten Aufnahmebereich für die Aufnahme der Messdaten umfasst.

Vorteilhaft kann aus dem bestimmten Aufnahmebereich ein Positionierungsparameter für die Messdatenaufnahmeeinheit für die Aufnahme von Messdaten des Aufnahmebereichs abgeleitet werden. Die Positionierungsparameter können weiterhin in Steuerungsbefehle für eine Positionierung der Messdatenaufnahmeeinheit mittels der Steuereinheit abgeleitet werden, worauf basierend das medizinische Bildgebungsgerät angesteuert wird. Vorteilhaft ist eine besonders zeiteffiziente und optimal abgestimmte Erzeugung des zweiten 3D Bilddatensatz ermöglicht. Vorteilhaft kann die Positionierung automatisiert oder zumindest teilautomatisiert werden und auf fehleranfällige und gegebenenfalls langwierige manuelle Positionierung verzichtet werden. Insgesamt kann dadurch vorteilhaft ein optimierter, und insbesondere automatisierter, Bildgebungsprozess und damit einen zeit- und dosiseffizienten Ablauf ermöglicht werden. Dabei kann insbesondere eine optimale Selektion des für eine Weiterführende Diagnose relevanten Bereichs miteingehen.

Das Positionieren einer Messdatenaufnahmeeinheit des medizinischen Bildgebungsgeräts relativ zum Patienten kann dabei das Positionieren der Messdatenaufnahmeeinheit selbst umfassen. Das Positionieren einer Messdatenaufnahmeeinheit des medizinischen Bildgebungsgeräts relativ zum Patienten kann dabei auch das Positionieren des Patienten mittels einer mittels der Steuereinheit ansteuerbaren, verfahrbaren Lagerungsvorrichtung, auf welcher der Patient für das Erzeugen des zweiten 3D Bilddatensatz gelagert ist und welche vom medizinischen Bildgebungsgerät umfasst ist, umfassen.

Der bestimmte Bildgebungsparameter einen Parameter die Bildrekonstruktion betreffend umfassen. Beispielsweise kann ein Rekonstruktionsvolumen, ein Parameter eines Rekonstruktionsverfahrens, ein Parameter betreffend eine von der Rekonstruktion umfassten Artefaktkorrektur oder auch ein anderweitiger Parameter bestimmt werden. Vorteilhaft kann eine auf den Zielbereich abgestimmte Rekonstruktion automatisch bereitgestellt werden.

Besonders bevorzugt umfasst das erfindungsgemäße Verfahren, dass der bestimmte Bildgebungsparameter ein Rekonstruktionsvolumen umfasst.

Vorteilhaft kann die Bildrekonstruktion des zweiten 3D Bilddatensatzes automatisiert oder zumindest teilautomatisiert durchgeführt werden und einen zeiteffizienten und optimierten Bildgebungsprozess ermöglichen. Vorteilhaft kann ein gegebenenfalls auf den Aufnahmebereich und/oder die Positionierung der Messdatenaufnahmeeinheit abgestimmtes Rekonstruktionsvolumen gewährleistet werden.

Eine insbesondere automatische Abstimmung der Bildakquisitions- und/oder Bildrekonstruktionsparameter basierend auf dem im ersten 3D Bilddatensatz identifizierten Zielbereich kann vorteilhaft einen optimierten Bildgebungsprozess und damit einen zeit- und dosiseffizienten Ablauf ermöglichen.

In einer Ausgestaltung des Verfahrens ist der erste dreidimensionale Bilddatensatz ein nativer Bilddatensatz, insbesondere nativer CT-Bilddatensatz, oder ein Angiographie-Bilddatensatz, insbesondere ein CT-Angiographie- Bilddatensatz, und der zweite Bilddatensatz ein Perfusions-Bilddatensatz, insbesondere ein CT-Perfusions-Bilddatensatz.

Ein CT-Perfusions-Bilddatensatz kommt häufig in sehr zeitkritischen Anwendungsfällen zum Einsatz, insbesondere bei einem Schlaganfallpatient. Besonders häufig wird dabei im Vorfeld eines CT-Perfusions-Bilddatensatzes bereits ein vorheriger Bilddatensatz, d.h. ein nativer CT-Bilddatensatz oder ein CT-Angiographie-Bilddatensatz, erzeugt. Häufig ist ein CT-Perfusions-Bilddatensatz, insbesondere beispielsweise im Rahmen eines mobilen medizinischen Bildgebungsgeräts, mit Einschränkungen hinsichtlich des abtastbaren Aufnahmebereichs verbunden. Vorteilhaft lässt sich durch das erfindungsgemäße Verfahren ein optimierter Bildgebungsprozess ermöglichen, welcher den möglicherweise eingeschränkten Aufnahmebereich optimal ausnutzt oder zumindest eine möglichst zeit- und dosiseffiziente Erzeugung des Perfusions-Bilddatensatz ermöglicht.

Weiterhin umfasst das erfindungsgemäße Verfahren, dass bei Vorliegen von einer Mehrzahl an ersten Bilddatensätzen, derjenige erste Bilddatensatz für das Identifizieren bereitgestellt wird, welcher den geringsten zeitlichen Abstand zum Zeitpunkt der Bereitstellung aufweist.

Liegt beispielsweise sowohl ein nativer CT-Bilddatensatz, ohne Zugabe von Kontrastmittel, als auch ein CT-Angiographie-Bilddatensatz vor, so wird der zuletzt aufgenommene Bilddatensatz für das Verfahren herangezogen werden und bereitgestellt werden. Vorteilhaft ist mit einer möglichst geringen Veränderung der Patientenposition, d.h. einer Bewegung des Untersuchungsbereichs, zwischen dem ersten und dem zweiten Bilddatensatz zu erwarten.

Die Erfindung betrifft außerdem eine Vorrichtung zur Ansteuerung eines medizinischen Bildgebungsgeräts, insbesondere eines mobilen medizinischen Bildgebungsgeräts, für das Erzeugen eines zweiten, 3D Bilddatensatzes umfassend zumindest einen Zielbereich eines Untersuchungsbereichs eines Patienten mit einer funktionellen Beeinträchtigung. Die erfindungsgemäße Vorrichtung umfasst eine Datenverarbeitungseinheit, ausgebildet einen ersten 3D Bilddatensatz bereitzustellen, den Zielbereich im ersten 3D Bilddatensatz zu identifizieren, wobei ein Teilbereich des Untersuchungsbereichs bestimmt wird, welcher von der funktionalen Beeinträchtigung betroffenen ist, einen Bildgebungsparameter basierend auf dem identifizierten Zielbereich für das Erzeugen des zweiten 3D Bilddatensatzes mittels des medizinischen Bildgebungsgeräts zu bestimmen. Die erfindungsgemäße Vorrichtung umfasst außerdem eine Steuerungseinheit, ausgebildet das medizinische Bildgebungsgerät basierend auf dem bestimmten Bildgebungsparameter für das Erzeugen des zweiten 3D Bilddatensatzes anzusteuern.

Eine erfindungsgemäße Vorrichtung zur Ansteuerung eines medizinischen Bildgebungsgeräts kann insbesondere dazu ausgebildet sein die zuvor beschriebenen erfindungsgemäßen Verfahren und ihre Aspekte auszuführen. Die Vorrichtung zur Ansteuerung eines medizinischen Bildgebungsgeräts kann dazu ausgebildet sein die Verfahren und ihre Aspekte auszuführen, indem die Datenverarbeitungseinheit und die Steuerungseinheit ausgebildet sind, die entsprechenden Verfahrensschritte auszuführen.

Die Ausgestaltungsvarianten, Merkmale und Vorteile des Verfahrens und seiner Ausführungsformen können dabei ebenso direkt auf die Vorrichtung übertragen werden.

In einer bevorzugten Vorrichtungsvariante ist das medizinische Bildgebungsgerät insbesondere ein CT-Gerät. Das CT-Gerät kann dabei insbesondere ausgebildet ein neben nativen CT-Bilddatensätzen, Angiographie-CT-Bilddatensätze und Perfusions-CT-Bilddatensätze zu erzeugen.

Die Erfindung betrifft weiterhin ein Fahrzeug, insbesondere einen Krankenwagen, welches ein medizinisches Bildgebungsgerät und eine erfindungsgemäße Vorrichtung umfasst.

Die Ausgestaltungsvarianten, Merkmale und Vorteile des Verfahrens und seiner Ausführungsformen können dabei ebenso direkt auf das Fahrzeug übertragen werden.

Ferner wird ein Computerprogrammprodukt vorgeschlagen, das ein Computerprogramm umfasst und direkt in einen Speicher einer Datenverarbeitungseinheit ladbar ist und Programmmittel, z.B. Bibliotheken und Hilfsfunktionen, aufweist, um ein Verfahren zur Ansteuerung eines medizinischen Bildgebungsgeräts auszuführen, wenn das Computerprogrammprodukt ausgeführt wird. Das Computerprogrammprodukt kann dabei eine Software mit einem Quellcode, der noch kompiliert und gebunden oder der nur interpretiert werden muss, oder einen ausführbaren Softwarecode umfassen, der zur Ausführung nur noch in die Datenverarbeitungseinheit zu laden ist. Durch das Computerprogrammprodukt kann das Verfahren zur Ansteuerung eines medizinischen Bildgebungsgeräts schnell, identisch wiederholbar und robust ausgeführt werden. Das Computerprogrammprodukt ist so konfiguriert, dass es mittels der Datenverarbeitungseinheit die erfindungsgemäßen Verfahrensschritte ausführen kann. Die Datenverarbeitungseinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können.

Das Computerprogrammprodukt ist beispielsweise auf einem computerlesbaren Medium gespeichert oder auf einem Netzwerk oder Server hinterlegt, von wo es in den Prozessor einer Datenverarbeitungseinheit geladen werden kann, der mit der Datenverarbeitungseinheit direkt verbunden oder als Teil der Datenverarbeitungseinheit ausgebildet sein kann. Weiterhin können Programmmittel des Computerprogrammprodukts auf einem elektronisch lesbaren Datenträger gespeichert sein. Die Programmmittel des elektronisch lesbaren Datenträgers können derart ausgestaltet sein, dass sie bei Verwendung des Datenträgers in einer Verarbeitungseinheit ein erfindungsgemäßes Verfahren durchführen. Beispiele für elektronisch lesbare Datenträger sind eine DVD, ein Magnetband oder ein USB-Stick, auf welchem elektronisch lesbare Programmmittel, insbesondere Software, gespeichert ist. Wenn diese Programmmittel von dem Datenträger gelesen und in eine Verarbeitungseinheit gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen.

Eine weitgehend softwaremäßige Realisierung hat den Vorteil, dass auch schon bisher verwendete Vorrichtungen zur Ansteuerung eines medizinischen Bildgebungsgeräts auf einfache Weise durch ein Software-Update nachgerüstet werden können, um auf die erfindungsgemäße Weise zu arbeiten. Ein solches Computerprogrammprodukt kann neben dem Computerprogramm gegebenenfalls zusätzliche Bestandteile, wie z.B. eine Dokumentation und/oder zusätzliche Komponenten, sowie Hardware-Komponenten, wie z.B. Hardware-Schlüssel (Dongles etc.) zur Nutzung der Software, umfassen.

Im Folgenden wird die Erfindung anhand von beispielhaften Ausführungsformen unter Hinweis auf die beigefügten Figuren erläutert. Die Darstellung in den Figuren ist schematisch, stark vereinfacht und nicht zwingend maßstabsgetreu. Es zeigen:
Fig. 1 ein erfindungsgemäßes Verfahren zur Ansteuerung eines medizinischen Bildgebungsgeräts,
Fig. 2 eine schematische Darstellung eines Längsschnittschnitts durch einen 3D Bilddatensatz,
Fig. 3 eine weitere, schematische Darstellung eines Querschnitts durch einen 3D Bilddatensatz,
Fig. 4 ein Fahrzeug umfassend ein medizinisches Bildgebungsgerät und eine erfindungsgemäße Vorrichtung.

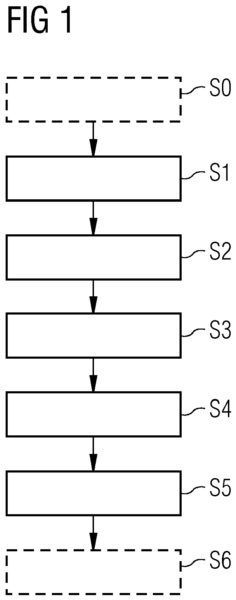

In Fig. 1 ist ein beispielhafter, schematischer Ablauf eines Verfahrens zur Ansteuerung eines medizinischen Bildgebungsgeräts 101, insbesondere eines mobilen medizinischen Bildgebungsgeräts, mittels einer Steuerungseinheit 113 für das Erzeugen eines zweiten dreidimensionalen Bilddatensatzes, welcher einen Zielbereich 11 in einem Untersuchungsbereich 3 eines Patienten 103 mit einer funktionellen Beeinträchtigung umfasst, dargestellt.

In einem Schritt des Bereitstellens S1 wird ein erster dreidimensionaler Bilddatensatzes umfassend den Untersuchungsbereich 3 des Patienten 103 mittels einer Datenverarbeitungseinheit 115 bereitgestellt.

In einem nächsten Schritt des Identifizierens S2 wird der Zielbereichs 11 basierend auf dem ersten dreidimensionalen Bilddatensatz mittels der Datenverarbeitungseinheit 115 identifiziert, wobei ein Teilbereich des Untersuchungsbereichs 3 bestimmt wird, welcher die funktionelle Beeinträchtigung aufweist.

In einer Variante des Verfahrens umfasst dabei der Untersuchungsbereich 3 zumindest einen Teil des Kopfes des Patienten 103 und der Zielbereich 11 zumindest einen Teil des Gehirns 13 des Patienten 103.

Insbesondere kann es sich bei dem Patienten 103 um einen Schlaganfallpatient, wobei im Schritt des Identifizierens S2 des Zielbereichs 11 als Teilbereich ein Teil 8,9 des Gehirns 13 des Patienten 103 bestimmt wird, welcher von einem ischämischen Infarkt betroffen ist.

Insbesondere kann dabei der ischämische Kernbereich 9 selbst bestimmt werden.

Der Zielbereich 11 kann dabei basierend auf einem Segmentationsalgorithmus, einem Schwellwertverfahrens und/oder mit Hilfe eines anatomischen Atlas und insbesondere automatisch basierend auf dem ersten dreidimensionalen Bilddatensatz identifiziert werden.

Insbesondere kann der Schritt des Identifizierens S2 das Anwenden einer trainierten Funktion umfassen.

In einem weiteren Schritt des Bestimmens S3 wird ein Bildgebungsparameter 4 für das Erzeugen des zweiten dreidimensionalen Bilddatensatzes basierend auf dem identifizierten Zielbereich 11 mittels der Datenverarbeitungseinheit 115 bestimmt.

Dabei kann der Bildgebungsparameter 4 ein Bildakquisitionsparameter 6,7 für die Aufnahme von Messdaten für das Erzeugen des zweiten dreidimensionalen Bilddatensatzes und/oder ein Bildrekonstruktionsparameter 5 für die Bildrekonstruktion des zweiten dreidimensionalen Bilddatensatzes sein.

Insbesondere kann der Bildgebungsparameter 4 ein Aufnahmebereich 6,7 und/oder ein Rekonstruktionsvolumen 5 sein.

In einem weiteren Schritt des Ansteuerns S5 wird das medizinische Bildgebungsgerät 101 basierend auf dem bestimmten Bildgebungsparameter 4 für die Erzeugung des zweiten dreidimensionalen Bilddatensatzes mittels der Steuerungseinheit 113 angesteuert.

Beispielsweise ist der bestimmte Bildgebungsparameter 4 ein Aufnahmebereich 6,7 für die Aufnahme von Messdaten mittels des medizinischen Bildgebungsgeräts 101 für das Erzeugen des zweiten dreidimensionalen Bilddatensatzes und das Ansteuern ein Positionieren einer Messdatenaufnahmeeinheit 102,104 des medizinischen Bildgebungsgeräts 101 relativ zu dem Patienten 103.

Das Verfahren kann ferner einen Schritt des ersten Erzeugens S0 des ersten 3D Bilddatensatz umfassen.

Das Verfahren kann ferner einen Schritt des zweiten Erzeugens S6 des zweiten 3D Bilddatensatzes im Rahmen der Ansteuerung des medizinischen Bildgebungsgeräts umfassen.

Insbesondere kann der erste dreidimensionale Bilddatensatz ein nativer Bilddatensatz oder ein Angiographie-Bilddatensatz sein, und der zweite dreidimensionale Bilddatensatz ein Perfusions-Bilddatensatz.

Erfindungsgemäß wird bei Vorliegen von einer Mehrzahl an ersten dreidimensionale Bilddatensätzen, vorteilhaft derjenige erste dreidimensionale Bilddatensatz bereitgestellt, welcher den geringsten zeitlichen Abstand zum Zeitpunkt der Bereitstellung aufweist.

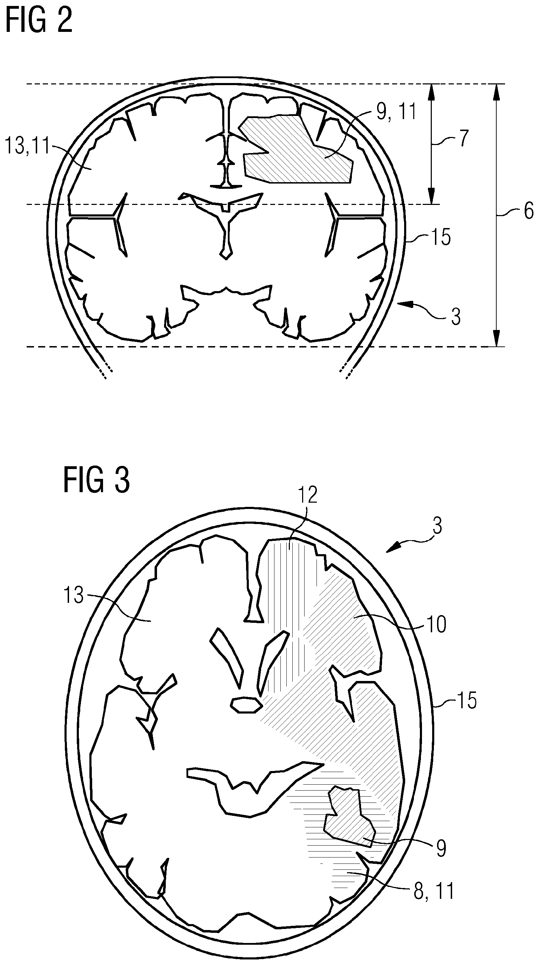

Fig. 2 und Fig. 3 zeigen zur Veranschaulichung eines möglichen Zielbereichs 11 jeweils eine schematische Darstellung eines Längsschnittschnitts bzw. eines Querschnitts durch einen ersten 3D Bilddatensatz. Ein solcher Schnitt kann im Wesentlichen einem Schichtbilddatensatz des ersten 3D Bilddatensatzes entsprechen.

Hier dargestellt sind lediglich Schnittbilddatensätze eines ersten 3D Bilddatensatzes. Die Identifizierung des Zielbereich 11 und Bestimmung des Bildgebungsparameters 6,7 kann jedoch ohne Weiteres auch auf einer räumlich dreidimensionalen Darstellung des ersten 3D Bilddatensatzes übertragen werden.

Der von den in den Fig. 2 und 3 beispielhaft dargestellten, ersten 3D Bilddatensätzen umfasste Untersuchungsbereich 3 ist dabei insbesondere zumindest ein Teil des Kopfes 3 eines Schlaganfallpatienten 103 mit einer funktionellen Beeinträchtigung durch einen ischämischen Infarkt. Angedeutet ist jeweils der Schädelknochen 15 und das Gehirn 13 des Patienten 103 im jeweils dargestellten Schnittbild des ersten 3D Bilddatensatz. Für die in den Figuren 2 und 3 dargestellten beispielhaften Bilddatensätze wird jeweils angenommen, dass im Bereich 9 ein Perfusionsdefizit durch den ischämischen Infarkt vorliegt. Insbesondere entspricht der Bereich 9 in den gezeigten Beispielen im Wesentlichen jeweils dem ischämischen Kernbereich.

Im Schritt des Identifizierens wird gemäß dem erfindungsgemäßen Verfahren basierend auf dem ersten 3D Bilddatensatz mittels der Datenverarbeitungseinheit 115 der Zielbereichs 11 identifiziert, wobei ein Teilbereich des Untersuchungsbereichs 3 bestimmt wird, welcher die funktionelle Beeinträchtigung aufweist. Das kann im Wesentlichen umfassen, die Bildbereiche im ersten 3D Bilddatensatz zu identifizieren, welche mit der funktionellen Beeinträchtigung verknüpft werden können.

In Fig. 2 ist beispielhaft dargestellt, dass der Zielbereich 11 das gesamte Gehirn 13 des Patienten 103 als Teilbereich des Kopfes 3, welcher die funktionelle Beeinträchtigung aufweist, umfassen kann. Beispielsweise kann das Gehirn 13 des Patienten dabei im Schritt des Identifizierens basierend auf den Bildwerten im ersten 3D Bilddatensatz mittels eines Schwellwertverfahrens, eines Segmentationsverfahren oder mittels einer trainierten Funktion identifiziert bzw. lokalisiert werden. Darauf basierend kann beispielsweise ein optimierter Aufnahmebereich 6 und/oder ein optimiertes Rekonstruktionsvolumen 6 umfassend den Bereich des Gehirns 13 des Patienten 103 bestimmt werden. Ausgehend von einem bestimmten Aufnahmebereich 6 kann weiterhin beispielsweise ein Positionierungsparameter basierend auf dem Aufnahmebereich 6 für das medizinische Bildgebungsgerät 101, für das Erzeugen des zweiten 3D Bilddatensatz bestimmt werden, welcher anschließend im Schritt des Ansteuerns mittels der Steuerungseinheit 115 für das Erzeugen des zweiten 3D Bilddatensatz angewendet wird.

Wie in Fig. 2 alternativ dargestellt kann auch lediglich ein Teil des Gehirns 13, welcher von der funktionellen Beeinträchtigung, hier durch den ischämischen Infarkt, betroffen ist, als Teilbereich bestimmt werden und damit vom identifizierten Zielbereich 11 umfasst sein. Der ischämische Kernbereich 9 kann gegebenenfalls direkt basierend auf dem ersten 3D Bilddatensatz identifiziert werden und als Zielbereich 11 bestimmt werden. Beispielsweise weichen die Bildwerte im ischämischen Kernbereich von denjenigen Bildwerten abseits des ischämischen Kernbereichs ab. Beispielsweise kann der ischämische Kernbereich 9 basierend auf einem Segmentationsalgorithmus oder einem Schwellwertverfahren automatisch im ersten 3D Bilddatensatz identifiziert werden. Es kann dafür beispielsweise auch der Einsatz eines maschinellen Lernverfahrens, d.h. das Anwenden einer trainierten Funktion, vorgesehen sein. Basierend auf dem ischämischen Kernbereich kann wiederum ein Bildgebungsparameter in Form eines Aufnahmebereichs 7 oder Rekonstruktionsvolumens 7 bestimmt werden, welches auf den Zielbereich abgestimmt ist. In diesem Fall kann der Aufnahmebereich 7 auf einen Teil des Gehirns 13 des Patienten 103 reduziert werden, womit vorteilhaft Dosis gespart werden kann und gegebenenfalls Einschränkungen des medizinischen Bildgebungsgeräts 101 hinsichtlich eines Aufnahmebereichs verbessert Rechnung getragen werden kann.

Alternativ dazu kann das Gehirn 13 eines Patienten 103, wie in Fig. 3 beispielhaft und nur schematisch dargestellt, im Schritt des Identifizierens auch basierend auf dem ersten 3D Bilddatensatz zuerst in mehrere Gehirnareale 8, 10, 12 unterteilt werden. Die Gehirnareale 8, 10, 12 können beispielsweise, müssen jedoch nicht notwendigerweise, mit bestimmten Körperfunktionen verknüpft sein. Beispielsweise kann dann ein Gehirnareal 8 als von der funktionellen Beeinträchtigung betroffen bewertet werden und als Zielbereich 11 identifiziert werden. Das Gehirnareal 8 kann als Zielbereich 11 identifiziert werden, indem die auftretenden Bildwerte oder auftretende Strukturen im ersten 3D Bilddatensatz bzw. jeweils gesondert in den Gehirnarealen 8, 10, 12 analysiert oder weiterverarbeitet werden. Das Gehirnareal 8 kann beispielsweise durch das Auftreten des Perfusionsdefizits in diesem Areal einen durchschnittlichen Bildwert aufweisen, welcher von einem Erwartungswert abweicht. Ein erwarteter Bildwert kann dabei basierend auf nicht betroffenen Gehirnarealen des Patienten 103, beispielsweise durch einen Vergleich der rechten und linken Hemisphäre des Gehirns 13, oder auch auf Erfahrungswerten aus bereits zuvor aufgenommenen Bilddatensätzen des Patienten 103 selbst oder einer Vielzahl von Patienten beruhen.

Ein Gehirnareal 8,10,12 kann auch anderweitig als von der funktionellen Beeinträchtigung betroffener Teilbereich bestimmt werden, beispielsweise wenn lokal oder global Bildwerte, örtlich zusammenhängende Gruppen von Bildwerten, oder anderweitige Strukturen, beispielsweise Schwankungen zwischen den auftretenden Bildwerten, auftreten, welche mit einer funktionellen Beeinträchtigung verknüpft werden können. Beispielsweise kann durch Anwenden einer trainierten Funktion ein Identifizieren eines betroffenen Gehirnareals zeiteffizient und sicher gewährleistet werden.

Auch in diesem Fall kann vorteilhaft ein, hier nicht weiter dargestellter, Aufnahmebereich und/oder ein Rekonstruktionsvolumen bestimmt werden, welcher auf einen Teil des Gehirns umfassend das Areal 8, eingeschränkt sein kann, womit vorteilhaft Dosis gespart werden kann und gegebenenfalls Einschränkungen des medizinischen Bildgebungsgeräts hinsichtlich eines Aufnahmebereichs verbessert Rechnung getragen werden kann.

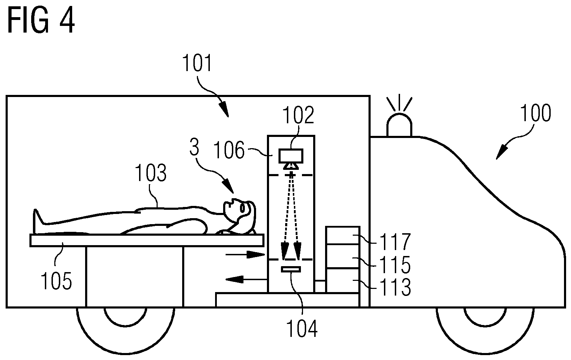

Fig. 4 zeigt ein Fahrzeug, insbesondere einen Krankenwagen, umfassend ein medizinisches Bildgebungsgerät 101 und eine Vorrichtung zur Ansteuerung des medizinischen Bildgebungsgeräts 101 für das Erzeugen eines zweiten dreidimensionalen Bilddatensatzes umfassend zumindest einen Zielbereich 11 in einem Untersuchungsbereich eines Patienten 103, insbesondere eines Schlaganfallpatienten, mit einer funktionellen Beeinträchtigung.

In diesem Beispiel ist das medizinische Bildgebungsgerät 101 ein Computertomographie-Gerät ist mit einer Messdatenaufnahmeeinheit 102,104 umfassend einen Röntgendetektor 104 und in Gegenüberstellung eine Röntgenquelle 102, welche in einer Gantry 106 angeordnet sind, welche eine Rotation der Messdatenaufnahmeeinheit 102,104 um eine gemeinsame Achse und damit die Aufnahme von Messdaten, d.h. insbesondere Röntgen-Projektionsmessdaten, eines Patienten 103 aus unterschiedlichen Winkelbereichen ermöglicht. Basierend auf diesen Messdaten kann dann ein erster oder zweiter dreidimensionaler Bilddatensatz, z.B. mittels eines Rekonstruktionsalgorithmus der gefilterten Rückprojektion, oder entsprechende Schichtbilddatensätze rekonstruiert werden.

Der Patient 103 ist dabei auf einer Patientenlagerungsvorrichtung 105 des medizinischen Bildgebungsgeräts 101 gelagert. Für die Aufnahme der Messdaten wird die Messdatenaufnahmeeinheit 102, 104 relativ zum Patienten positioniert, so dass ein bestimmter Aufnahmebereich 6,7 mittels der Messdatenaufnahmeeinheit 102,104 abgetastet werden kann. Eine Positionierung, exemplarisch angedeutet durch die Pfeile, kann durch ein Verfahren oder Positionieren der Patientenlagerungsvorrichtung 105 und/oder auch durch ein Verfahren oder Positionieren der Messdatenaufnahmeeinheit 102,104, d.h. im Wesentlichen der Gantry 106, ermöglicht werden.

Die Vorrichtung zur Ansteuerung des medizinischen Bildgebungsgeräts 101 weist insbesondere eine Datenverarbeitungseinheit 115 auf, welche ausgebildet ist, einen ersten dreidimensionalen Bilddatensatz umfassend den Untersuchungsbereich 3 des Patienten 103 bereitzustellen, den Zielbereich 11 basierend auf dem ersten dreidimensionalen Bilddatensatz zu identifizieren, wobei ein Teilbereich des Untersuchungsbereichs bestimmt wird, welcher die funktionelle Beeinträchtigung aufweist, und einen Bildgebungsparameter 4 basierend auf dem identifizierten Zielbereich 11 für das Erzeugen des zweiten dreidimensionalen Bilddatensatzes zu bestimmen.

Die Vorrichtung weißt außerdem eine Steuerungseinheit 113 auf, welche ausgebildet ist, das medizinische Bildgebungsgerät 101 basierend auf dem bestimmten Bildgebungsparameter 4 für das Erzeugen des zweiten dreidimensionalen Bilddatensatzes anzusteuern.

Die Datenverarbeitungseinheit 115 und die Steuerungseinheit 113 kann in Form eines Computers, eines Mikrocontrollers oder eines integrierten Schaltkreises umgesetzt sein. Die Datenverarbeitungseinheit 115 und die Steuerungseinheit 113 kann Hardware-Elemente oder Software-Elemente aufweisen, beispielsweise einen Mikroprozessor oder ein sogenanntes FPGA (englisches Akronym für "Field Programmable Gate Array"). Es kann sich auch um einen Verbund von Computern handeln (ein englischer Fachbegriff für einen realen Verbund ist "Cluster")

Die Vorrichtung kann außerdem eine Speichereinheit 117 umfassen. Die Speichereinheit 117 kann auch von der Datenverarbeitungseinheit umfasst sein. Diese kann als nicht dauerhafte Arbeitsspeicher (Random Access Memory, kurz RAM) oder als dauerhafter Massen-speicher (Festplatte, USB-Stick, SD-Karte, Solid State Disk) realisiert sein. Bei einer Schnittstelle 15 kann es sich um eine Hardware- oder Softwareschnittstelle handeln (beispielsweise PCI-Bus, USB oder Firewire). Die Speichereinheit 117 kann dabei beispielsweise ausgelegt sein, einen ersten 3D Bilddatensatz für die Verwendung gemäß des erfindungsgemäßen Verfahrens zwischenzuspeichern.

Vorzugsweise weist die Vorrichtung weiterhin wenigstens eine, hier nicht dargestellte, Eingabeeinheit und/oder wenigstens eine Ausgabeeinheit auf. Eine Eingabeeinheit und/oder Ausgabeeinheit ermöglicht beispielsweise die manuelle Interaktion eines Anwenders, beispielsweise das Starten oder Stoppen des erfindungsgemäßen Verfahrens, eine Bestätigung oder eine Korrektur durch einen Anwender. Eine Ausgabeeinheit in Form einer Darstellungseinheit kann außerdem das Darstellen des ersten und/oder zweiten dreidimensionalen Bilddatensatzes ermöglichen.

Der Gegenstand der Erfindung ist nicht auf die vorstehend beschriebenen Ausführungsbeispiele beschränkt. Vielmehr können weitere Ausführungsformen der Erfindung von dem Fachmann aus der vorstehenden Beschreibung abgeleitet werden. Insbesondere können die anhand der verschiedenen Ausführungsbeispiele beschriebenen Einzelmerkmale der Erfindung und deren Ausgestaltungsvarianten auch in anderer Weise miteinander kombiniert werden.

## Patentansprüche

1. Verfahren zur Ansteuerung eines medizinischen Bildgebungsgeräts (101) mittels einer Steuerungseinheit (113) für das Erzeugen eines zweiten dreidimensionalen Bilddatensatzes, welcher einen Zielbereich (11) in einem Untersuchungsbereich (3) eines Patienten (103) mit einer funktionellen Beeinträchtigung umfasst, aufweisend die Schritte
- Bereitstellen (S1) eines ersten dreidimensionalen Bilddatensatzes umfassend den Untersuchungsbereich (3) des Patienten (103) mittels einer Datenverarbeitungseinheit (115),
- Identifizieren (S2) des Zielbereichs (11) basierend auf dem ersten dreidimensionalen Bilddatensatz mittels der Datenverarbeitungseinheit (115), wobei ein Teilbereich des Untersuchungsbereichs (3) bestimmt wird, welcher die funktionelle Beeinträchtigung aufweist,
- Bestimmen (S3) eines Bildgebungsparameters (6,7) für das Erzeugen des zweiten dreidimensionalen Bilddatensatzes basierend auf dem identifizierten Zielbereich (11) mittels der Datenverarbeitungseinheit (115),
- Ansteuern (S5) des medizinischen Bildgebungsgeräts (101) basierend auf dem Bildgebungsparameter (6,7) für die Erzeugung des zweiten dreidimensionalen Bilddatensatzes mittels der Steuerungseinheit (113)
- wobei bei Vorliegen von einer Mehrzahl an ersten dreidimensionale Bilddatensätzen, derjenige erste dreidimensionale Bilddatensatz für das Identifizieren bereitgestellt wird, dessen Aufnahmezeitpunkt den geringsten zeitlichen Abstand zum Zeitpunkt der Bereitstellung aufweist.

2. Verfahren nach Anspruch 1, wobei der Untersuchungsbereich (3) zumindest einen Teil des Kopfes des Patienten (103) umfasst und wobei der Zielbereich (11) zumindest ein Teil des Gehirns (13) des Patienten (103) umfasst.

3. Verfahren nach einem der Ansprüche 1 bis 2, wobei der Patient (103) ein Schlaganfallpatient ist und im Schritt des Identifizierens (S2) als Teilbereich ein Teil (8,9) des Gehirns (13) des Patienten (103) bestimmt wird, welcher von einem ischämischen Infarkt betroffen ist.

4. Verfahren nach einem der Ansprüche 1 bis 3, wobei im Schritt des Identifizierens (S2) ein ischämischer Kernbereich (9) bestimmt wird.

5. Verfahren nach einem der Ansprüche 1 bis 4, wobei der Zielbereich (11) basierend auf einem Segmentationsalgorithmus, einem Schwellwertverfahrens und/oder mit Hilfe eines anatomischen Atlas automatisch basierend auf dem ersten dreidimensionalen Bilddatensatz identifiziert wird.

6. Verfahren nach einem der Ansprüche 1 bis 5, wobei der Schritt des Identifizierens (S2) das Anwenden einer trainierten Funktion umfasst.

7. Verfahren nach einem der Ansprüche 1 bis 6, wobei der Bildgebungsparameter (6,7) ein Bildakquisitionsparameter für die Aufnahme von Messdaten für das Erzeugen des zweiten dreidimensionalen Bilddatensatzes und/oder ein Bildrekonstruktionsparameter für die Bildrekonstruktion des zweiten dreidimensionalen Bilddatensatzes umfasst.

8. Verfahren nach einem der Ansprüche 1 bis 7, wobei der Bildgebungsparameter (6,7) ein Aufnahmebereich und/oder ein Rekonstruktionsvolumen umfasst.

9. Verfahren nach einem der Ansprüche 1 bis 7, wobei der Bildgebungsparameter (6,7) ein Aufnahmebereich für die Aufnahme von Messdaten mittels des medizinischen Bildgebungsgeräts (101) für das Erzeugen des zweiten dreidimensionalen Bilddatensatzes umfasst und wobei das Ansteuern ein Positionieren einer Messdatenaufnahmeeinheit (102,104) des medizinischen Bildgebungsgeräts (101) relativ zu dem Patienten (103) umfasst.

10. Verfahren nach einem der Ansprüche 1 bis 9, wobei der erste dreidimensionale Bilddatensatz ein nativer Bilddatensatz oder ein Angiographie-Bilddatensatz ist, und wobei der zweite dreidimensionale Bilddatensatz ein Perfusions-Bilddatensatz ist.

11. Vorrichtung zur Ansteuerung eines medizinischen Bildgebungsgeräts (101) für das Erzeugen eines zweiten dreidimensionalen Bilddatensatzes umfassend zumindest einen Zielbereich (11) in einem Untersuchungsbereich eines Patienten (103) mit einer funktionellen Beeinträchtigung, aufweisend
- eine Datenverarbeitungseinheit (115), ausgebildet
• einen ersten dreidimensionalen Bilddatensatz umfassend den Untersuchungsbereich (3) des Patienten (103) bereitzustellen,
• den Zielbereich (11) basierend auf dem ersten dreidimensionalen Bilddatensatz zu identifizieren, wobei ein Teilbereich des Untersuchungsbereichs (3) bestimmt wird, welcher die funktionelle Beeinträchtigung aufweist, und
• einen Bildgebungsparameter (6,7) basierend auf dem identifizierten Zielbereich (11) für das Erzeugen des zweiten dreidimensionalen Bilddatensatzes zu bestimmen, und
• bei Vorliegen von einer Mehrzahl an ersten dreidimensionale Bilddatensätzen, denjenigen ersten dreidimensionalen Bilddatensatz für das Identifizieren bereitzustellen, dessen Aufnahmezeitpunkt den geringsten zeitlichen Abstand zum Zeitpunkt der Bereitstellung aufweist,
- eine Steuerungseinheit (113), ausgebildet das medizinische Bildgebungsgerät (101) basierend auf dem bestimmten Bildgebungsparameter (6,7) für das Erzeugen des zweiten dreidimensionalen Bilddatensatzes anzusteuern.

12. Vorrichtung nach Anspruch 11, wobei das medizinisches Bildgebungsgerät (101) ein Computertomographie-Gerät ist.

13. Vorrichtung nach einem der Ansprüche 11 oder 12, außerdem ausgebildet ein Verfahren gemäß den Ansprüchen 2 bis 10 durchzuführen.

14. Fahrzeug (100), insbesondere Krankenwagen, umfassend ein medizinisches Bildgebungsgerät (101) und eine Vorrichtung gemäß einem der Ansprüche 11 bis 13.

15. Computerprogrammprodukt umfassend ein Computerprogramm, welches direkt in einen Speicher (117) einer Datenverarbeitungseinheit (115) ladbar ist, mit Programmmitteln, um ein Verfahren zur Ansteuerung eines medizinischen Bildgebungsgeräts (101) nach einem der Ansprüche 1 bis 10 auszuführen, wenn das Computerprogrammprodukt durch die Datenverarbeitungseinheit (115) ausgeführt wird.

## Claims

1. Method for actuating a medical imaging device (101) by means of a control unit (113) for generating a second three-dimensional image dataset which includes a target region (11) in a region of interest (3) of a patient (103) with a functional impairment, with the steps
- providing (S1) a first three-dimensional image dataset including the region of interest (3) of the patient (103) by means of a data processing unit (115),
- identifying (S2) the target region (11) based on the first three-dimensional image dataset by means of the data processing unit (115), wherein a partial region of the region of interest (3) with the functional impairment is determined,
- determining (S3) an imaging parameter (6,7) for generating the second three-dimensional image dataset based on the identified target region (11) by means of the data processing unit (115),
- actuating (S5) the medical imaging device (101) based on the imaging parameter (6,7) for the generation of the second three-dimensional image dataset by means of the control unit (113),
- wherein in the case of a plurality of first three-dimensional image datasets, the first three-dimensional image dataset whose recording time has the shortest time interval to the time of the provision is provided for the identification.

2. Method according to claim 1, wherein the region of interest (3) includes at least one part of the head of the patient (103) and wherein the target region (11) includes at least one part of the brain (13) of the patient (103).

3. Method according to one of claims 1 to 2, wherein the patient (103) is a stroke patient and in the identifying step (S2) a part (8,9) of the brain (13) of the patient (103) which is affected by an ischaemic infarction is determined as a partial region.

4. Method according to one of claims 1 to 3, wherein an ischemic core region (9) is determined in the identifying step (S2) .

5. Method according to one of claims 1 to 4, wherein the target region (11) is identified based on a segmentation algorithm, a threshold method and/or with the aid of an anatomical atlas automatically based on the first three-dimensional image dataset.

6. Method according to one of claims 1 to 5, wherein the identifying step (S2) includes the application of a trained function.

7. Method according to one of claims 1 to 6, wherein the imaging parameter (6,7) includes an image acquisition parameter for recording of measurement data for generating the second three-dimensional image dataset and/or an image reconstruction parameter for image reconstruction of the second three-dimensional image dataset.

8. Method according to one of claims 1 to 7, wherein the imaging parameter (6,7) includes a recording region and/or a reconstruction volume.

9. Method according to one of claims 1 to 7, wherein the imaging parameter (6,7) includes a recording region for the recording of measurement data by means of the medical imaging device (101) for generating the second three-dimensional image dataset and wherein the actuation includes positioning a measurement data recording unit (102,104) of the imaging device (101) relative to the patient (103).

10. Method according to one of claims 1 to 9, wherein the first three-dimensional image dataset is a native image dataset or an angiography image dataset and wherein the second three-dimensional image dataset is a perfusion image dataset.

11. Apparatus for actuating a medical imaging device (101) for generating a second three-dimensional image dataset including at least one target region (11) in a region of interest of a patient (103) with a functional impairment, with
- a data processing unit (115) embodied
• to provide a first three-dimensional image dataset including the region of interest (3) of the patient (103),
• to identify the target region (11) based on the first three-dimensional image dataset, wherein a partial region of the region of interest (3) with the functional impairment is determined and
• to determine an imaging parameter (6,7) based on the identified target region (11) for generating the second three-dimensional image dataset, and
• in the case of a plurality of first three-dimensional image datasets, to provide the first three-dimensional image dataset whose recording time has the shortest time interval to the time of the provision for the identification,
- a control unit (113) embodied to actuate the medical imaging device (101) based on the determined imaging parameter (6,7) for generating the second three-dimensional image dataset.

12. Apparatus according to claim 11, wherein the medical imaging device (101) is a computed tomography device.

13. Apparatus according to one of claims 11 or 12 additionally embodied to carry out a method according to the claims 2 to 10.

14. Vehicle (100), in particular an ambulance, including a medical imaging device (101) and an apparatus according to one of claims 11 to 13.

15. Computer program product including a computer program, which can be loaded directly into a memory (117) of a data processing unit (115), with program means for executing a method for actuating a medical imaging device (101) according to one of claims 1 to 10 when the computer program product is executed by the data processing unit (115).

## Revendications

1. Procédé de commande d'un appareil (101) d'imagerie médicale au moyen d'une unité (113) de commande pour la production d'un deuxième ensemble de données d'image en trois dimensions, qui comprend un domaine (11) cible dans un domaine (3) à examiner d'un patient (103) ayant une altération fonctionnelle, comportant les stades
- on se procure (S1) un premier ensemble de données d'image en trois dimensions comprenant le domaine (3) à examiner du patient (103) au moyen d'une unité (115) de traitement de données,
- on identifie (S2) le domaine (11) cible en se fondant sur le premier ensemble de données d'image en trois dimensions au moyen de l'unité (115) de traitement de données, dans lequel on détermine, du domaine (3) à examiner, un domaine partiel, qui comporte l'altération fonctionnelle,
- on détermine (S3) un paramètre (6, 7) d'imagerie pour la production du deuxième ensemble de données d'image en trois dimensions reposant sur le domaine (11) cible identifié au moyen de l'unité (115) de traitement de données,
- on commande (S5) l'appareil (101) d'imagerie médicale sur la base du paramètre (6, 7) d'imagerie pour la production du deuxième ensemble de données d'image en trois dimensions au moyen de l'unité (113) de commande,
- dans lequel, en présence d'une pluralité de premiers ensembles de données d'image en trois dimensions, on prend pour l'identification le premier ensemble de données d'image en trois dimensions, dont l'instant d'enregistrement a la distance dans le temps la plus petite à l'instant où on le prend.

2. Procédé suivant la revendication 1, dans lequel le domaine (3) à examiner comprend au moins une partie de la tête du patient (103) et dans lequel le domaine (11) cible comprend au moins une partie du cerveau (13) du patient (103).

3. Procédé suivant l'une des revendications 1 à 2, dans lequel le patient (103) est un sujet ayant une attaque d'apoplexie et, dans le stade de l'identification (S2), on détermine comme domaine partiel une partie (8, 9) du cerveau (13) du patient (103), qui est atteinte de l'infarctus ischémié.

4. Procédé suivant l'une des revendications 1 à 3, dans lequel, dans le stade de l'identification (S2), on détermine un domaine (9) ischémié du noyau.

5. Procédé suivant l'une des revendications 1 à 4, dans lequel on identifie le domaine (11) cible, sur la base d'un algorithme de segmentation, d'un procédé à valeur de seuil et/ou à l'aide d'un atlas anatomique, d'une manière automatique en se fondant sur le premier ensemble de données d'image en trois dimensions.

6. Procédé suivant l'une des revendications 1 à 5, dans lequel le stade de l'identification (S2) comprend l'application d'une fonction d'apprentissage.

7. Procédé suivant l'une des revendications 1 à 6, dans lequel le paramètre (6, 7) d'imagerie comprend un paramètre d'acquisition d'image pour l'enregistrement de données de mesure pour la production du deuxième ensemble de données d'image en trois dimensions et/ou un paramètre de reconstruction d'image pour la reconstruction d'image du deuxième ensemble de données d'image en trois dimensions.

8. Procédé suivant l'une des revendications 1 à 7, dans lequel le paramètre (6, 7) d'imagerie comprend un domaine d'enregistrement et/ou un volume de reconstruction.

9. Procédé suivant l'une des revendications 1 à 7, dans lequel le paramètre (6, 7) d'imagerie comprend un domaine d'enregistrement pour l'enregistrement de données de mesure au moyen de l'appareil (101) d'imagerie médicale pour la production du deuxième ensemble de données d'image en trois dimensions, et dans lequel la commande comprend une mise en position d'une unité (102, 104) d'enregistrement de données de mesure de l'appareil (101) d'imagerie médicale par rapport au patient (103).

10. Procédé suivant l'une des revendications 1 à 9, dans lequel un premier ensemble de données d'image en trois dimensions est un ensemble de données d'image natif ou un ensemble de données d'image d'angiographie, et dans lequel le deuxième ensemble de données d'image en trois dimensions est un ensemble de données d'image de perfusion.

11. Dispositif de commande d'un appareil (101) d'imagerie médicale pour la production d'un deuxième ensemble de données d'image en trois dimensions comprenant au moins un domaine (11) cible dans un domaine à examiner d'un patient (103) ayant une altération fonctionnelle, comportant
- une unité (115) de traitement de données constituée
• pour disposer d'un premier ensemble de données d'image en trois dimensions comprenant le domaine (3) à examiner du patient (103),
• pour identifier le domaine (11) cible sur la base du premier ensemble de données d'image en trois dimensions, dans lequel on détermine, du domaine (3) à examiner, un domaine partiel, qui a l'altération fonctionnelle, et
• pour déterminer un paramètre (6, 7) d'imagerie reposant sur le domaine (11) cible identifié, pour la production du deuxième ensemble de données d'image en trois dimensions, et
• en présence d'une pluralité de premiers ensembles de données d'image en trois dimensions, pour prendre, pour l'identification, le premier ensemble de données d'image en trois dimensions, dont l'instant d'enregistrement a la distance dans le temps la plus petite à l'instant où le prend,
- une unité (113) de commande constituée pour commander l'appareil (101) d'imagerie médicale sur la base du paramètre (6, 7) d'imagerie déterminé pour la production du deuxième ensemble de données d'image en trois dimensions.

12. Dispositif suivant la revendication 11, dans lequel l'appareil (101) d'imagerie médicale est un appareil de tomodensitométrie assisté par ordinateur.

13. Dispositif suivant l'une des revendications 11 ou 12, constitué en outre pour exécuter un procédé suivant les revendications 2 à 10.

14. Véhicule (100), notamment ambulance, comprenant un appareil (101) d'imagerie médicale et un dispositif suivant l'une des revendications 11 à 13.

15. Produit de programme d'ordinateur comprenant un programme d'ordinateur, qui peut être chargé directement dans une mémoire (117) d'une unité (115) de traitement de données, comprenant des moyens de programme pour effectuer un procédé de commande d'un appareil (101) d'imagerie médicale suivant l'une des revendications 1 à 10, lorsque le produit de programme d'ordinateur est réalisé par l'unité (115) de traitement de données.
